# EUROPÄISCHE PATENTSCHRIFT

(11) **EP 3 073 262 B1**
(45) Veröffentlichungstag und Bekanntmachung des Hinweises auf die Patenterteilung: **13.07.2022**
(21) Anmeldenummer: 16161956.4
(22) Anmeldetag: 23.03.2016
(51) Int. Cl.: G01N 33/44, G01N 19/04

(54) **VORRICHTUNG UND VERFAHREN ZUR ERMITTLUNG DER KLEBRIGKEIT VORIMPRÄGNIERTER FASERHALBZEUGE**
DEVICE AND METHOD FOR DETERMINING THE TACKINESS OF PRE-IMPREGNATED SEMI-FINISHED FIBRE PRODUCTS
DISPOSITIF ET PROCÉDÉ DE DÉTERMINATION DE L'ADHÉRENCE DE DEMI-PRODUITS EN FIBRES PRÉ-IMPRÉGNÉS

(30) Priorität: 26.03.2015 DE 102015104612
(43) Veröffentlichungstag der Anmeldung: 28.09.2016
(73) Patentinhaber: Deutsches Zentrum für Luft- und Raumfahrt e.V., 51147 Köln (DE)
(72) Erfinder: NGUYEN, Duy Chinh, 21682 Stade (DE); GROHMANN, Yannis, 20249 Hamburg (DE); KROMBHOLZ, Christian, 21614 Buxtehude (DE)
(74) Vertreter: Aisch, Sebastian

(56) Entgegenhaltungen:
- EP-A1- 1 334 819
- DE-A1- 4 014 939
- JP-A- 2004 177 277
- US-A- 4 874 948
- US-A- 5 513 537

## Beschreibung

Die Erfindung betrifft ein Verfahren und eine Vorrichtung zur Ermittlung der Klebrigkeit vorimprägnierter Faserhalbzeuge zur Herstellung eines Faserverbundbauteils. Die Erfindung betrifft ebenso eine Vorrichtung und ein Verfahren zum Ablegen von Fasern auf einem Werkzeug zur Herstellung eines Faserverbundbauteils, bei dem die Klebrigkeit des abzulegenden Faserhalbzeuges ermittelt wird bzw. wurde.

Aufgrund der vorteilhaften Eigenschaften von Faserverbundwerkstoffen, bei einem relativ geringen Gewicht eine hohe Festigkeit und Steifigkeit in zumindest einer Richtung aufzuweisen, sind Faserverbundwerkstoffe aus der Luft und Raumfahrt nicht mehr wegzudenken. Aber auch im Automobilbereich werden Faserverbundwerkstoffe vermehrt eingesetzt, um bei gleichbleibender Steifigkeit und Festigkeit das Gewicht der Fahrzeuge zu reduzieren, was meist proportional mit der Einsparung von Treibstoff einhergeht.

Neben dem klassischen Verfahren, bei denen meist händisch das Fasermaterial auf einem Formwerkzeug drapiert und anschließend unter Temperaturbeaufschlagung sowie Bedruckung das Faserverbundbauteil ausgehärtet wird, treten mehr und mehr automatisierte Ablegeprozesse in den Vordergrund, um die Produktionskosten faserverstärkter Kunststoffbauteile zu reduzieren.

Für die Fertigung von Großbauteilen in der Fiber Placement beziehungsweise der Tape Laying Technologie ist beispielsweise aus der DE 10 2010 015 027 B1 eine Faserlegeanlage bekannt, mit der der Prozessschritt des Legens der Fasern auf dem Werkzeug größtenteils automatisiert wurde. Hierbei werden Roboter auf einem um das Werkzeug umlaufenden Schienensystem geführt, wobei die Roboter entsprechende Faserlegeköpfe als Endeffektoren aufweisen, mit denen das Fasermaterial auf dem Werkzeug abgelegt werden kann. Das Fasermaterial wird dabei dem Faserlegekopf mittels einer Zufuhreinrichtung zugeführt, die mit einer Fasermaterialbereitstellungseinrichtung beziehungsweise einem Fasermaterialspeicher kontaktiert ist.

Die hierbei einzustellenden Prozessparameter wie Kompaktierungskraft, Temperatur des Materials oder Ablegegeschwindigkeit, werden in der Regel für den verwendeten Materialtyp bereits im Vorfeld fest definiert und dann anhand der festgelegten Prozessparameter durchgeführt. Dabei hat die Klebrigkeit des Fasermaterials einen nicht unerheblichen Einfluss darauf, wie die Prozessparameter einzustellen sind.

Unter der Klebrigkeit des Fasermaterials wird dabei insbesondere der Tack beziehungsweise die Klebeigenschaft des Fasermaterials verstanden. Unter der Klebrigkeit des Fasermaterials im Sinne der vorliegenden Erfindung wird insbesondere die Adhäsion beziehungsweise die Adhäsionseigenschaften des Fasermaterials in Bezug auf andere Materialien, wie beispielsweise des Formwerkzeuges, verstanden.

Bei der Verarbeitung von Prepreg-Material wird in der Regel vom Materialhersteller eine Verarbeitungszeit (Tacklife) angegeben, in der das Material verarbeitet werden sollte. Nach Überschreiten der Tacklife (je nach Material beispielsweise 10 bis 15 Tage) hat die Klebrigkeit des Materials soweit abgenommen, dass eine Verarbeitung des Materials erschwert oder gar unmöglich wird. Denn bei einer zu niedrigen Klebrigkeit können die abgelegten Fasern beispielsweise nicht mehr auf der Werkzeugoberfläche haften oder mehrere Materiallagen nicht mehr prozesssicher verklebt werden. Darüber hinaus kann die Ablage von Material mit einer geringeren Klebrigkeit zu reduzierten mechanischen Eigenschaften des Bauteils führen.

Da die Klebrigkeit des Materials sich während der Verarbeitungszeit drastisch verändert und einen großen Einfluss auf den Ablegeprozess hat, ist es wünschenswert, die Prozessparameter an die Klebrigkeit des abzulegenden Fasermaterials anzupassen. Ist das Material beispielsweise relativ "frisch" und die Klebrigkeit dementsprechend hoch, kann mit einer angepassten Temperatur und/oder angepassten Anpresskraft und/oder angepassten Geschwindigkeit abgelegt werden. Ist die Klebrigkeit hingegen nicht mehr so hoch, könnte das Material trotzdem noch abgelegt werden, denn die Temperatur und/oder die Kontaktierungskraft und/oder die Geschwindigkeit entsprechend angepasst werden.

Aus der US 6,814,822 B2 ist ein Faserlegekopf zum Legen von Fasermaterial bekannt, bei dem während des Ablegens der Fasern die molekulare Mobilität des Harzes Mittels einer Magnetresonanzmethode ermittelt wird, wobei anhand der molekularen Mobilität dann auf die Klebrigkeit des Fasermaterials geschlossen wird. Dieses Verfahren kann dabei kontinuierlich während des Ablegeprozesses durchgeführt werden, wobei in Abhängigkeit der ermittelten Klebrigkeit dann gegebenenfalls andere Prozessparameter beeinflusst werden.

Nachteilig hierbei ist jedoch, dass die Klebrigkeit mit einem relativ großen Aufwand ermittelt wird, wobei bei der Anwendung einer Magnetresonanzmethode besondere Vorkehrungen insbesondere in Bezug auf den Untersuchungsraum getroffen werden müssen. Hierdurch wird der bauliche Aufwand des Ablegekopfes stark erhöht.

Aus der JP 2004-177277 A ist eine mechanische Messvorrichtung zum Ermitteln der Klebrigkeit von vorimprägnierten Fasermaterialien bekannt, wobei eine Fasermaterialprobe auf einen Schlitten gelegt und dann von oben mit einem Stempel druckbeaufschlagt wird, sodass das Fasermaterial zwischen dem Schlitten und dem Stempel eingeklemmt wird. Es wird nun versucht, den Schlitten in eine Richtung zu ziehen, um so eine relative Bewegung zwischen Fasermaterial und dem Stempel/Schlitten zu bewirken, sodass auf das Fasermaterial eine Scherkraft wirkt, die gemessen werden kann, um so auf die Klebrigkeit zu schließen.

Aus der DE 40 14 939 A1 ist eine mechanische Messvorrichtung bekannt, mit der fortlaufend die Klebrigkeit einer Schicht aus unvulkanisiertem Gummimaterial gemessen werden kann. Dabei wird eine Rolle mit dem Material in Verbindung gebracht, das dann in Förderrichtung bewegt wird, wodurch die Rolle in Förderrichtung mitgenommen wird, wobei sich die Kraft des Mitnehmens messen lässt und hieraus die Klebrigkeit ableiten lässt.

Aus der US 4 874 948 A ist eine Vorrichtung zum Ermitteln des Aushärtungsgrades von vorimprägnierten Fasermaterialien bekannt, der sich aus der Temperaturverteilung während des Aushärtens ableiten lässt.

Aus der US 5 513 537 A ist schließlich ein Verfahren zum Ermitteln der Klebrigkeit von Fasermaterialien bekannt, wobei hier mehrere Prozessschritte durchgeführt werden. Einer der Prozessschritte sieht dabei unter anderem vor, eine Materialprobe mit einer transluzenten Scheibe abzudecken und dann das Ganze zu beleuchten und aus einem anderen Winkel aufzunehmen, wodurch sich eine Graustufenverteilung im aufgenommen Bild ergibt. Hieraus soll dann die Klebrigkeit abgeleitet werden.

Es ist daher Aufgabe der vorliegenden Erfindung eine verbesserte Vorrichtung und ein verbessertes Verfahren anzugeben, mit dem die Klebrigkeit des Fasermaterials sowohl diskret als auch kontinuierlich einfach und sicher bestimmt werden kann.

Die Aufgabe wird mit der Ermittlungseinrichtung gemäß Anspruch 1 sowie dem Ermittlungsverfahren gemäß Anspruch 10 erfindungsgemäß gelöst.

Demnach ist erfindungsgemäß eine Ermittlungseinrichtung vorgesehen, die eine mechanische Messeinheit aufweist.

Die mechanische Messeinheit weist hierbei ein Reibelement auf, das eine gekrümmte Oberfläche hat. Das Reibelement der mechanischen Messeinheit ist dabei so ausgebildet, dass es mit einer vorgegebenen Kraft mit der gekrümmten Oberfläche auf ein aufgespanntes Fasermaterial aufdrückbar ist. So kann das Reibelement beispielsweise bewegbar gelagert sein, so dass es mittels eines Linearmotors in Richtung Fasermaterial verfahrbar ist und mit der vorgegebenen Kraft auf das aufgespannte Fasermaterial aufgedrückt wird.

Ist das Reibelement mit der gekrümmten Oberfläche auf das aufgespannte Fasermaterial aufgedrückt, so kontaktiert die gekrümmte Oberfläche das Fasermaterial an einer vorgegebenen Länge, wobei in dem Kontaktbereich zwischen der gekrümmten Oberfläche des Reibelementes und des Fasermaterials eine entsprechende Haftreibung aufgrund der vorgegebenen Andruckkraft entsteht.

Die mechanische Messeinheit ist nun so ausgebildet, dass sie eine Scherkraft zwischen dem Fasermaterial und dem aufgedrückten Reibelement aufbringt, wobei die Klebrigkeit des kontaktierten Fasermaterials dann in Abhängigkeit von der benötigten Scherkraft, bei dem die Haftreibung zwischen dem Fasermaterial und der gekrümmten Oberfläche des Reibelementes in Gleitreibung übergeht, ermittelt wird.

In anderen Worten, die mechanische Messeinheit bringt eine Scherkraft zwischen dem Fasermaterial und der gekrümmten Oberfläche auf und steigert dabei die Scherkraft so lange, bis die Haftreibung zwischen Fasermaterial und gekrümmter Oberfläche des Reibelementes in die Gleitreibung übergeht, wobei der Übergang beispielsweise mithilfe eines Sensors detektierbar ist und die Klebrigkeit des Fasermaterials dann in Abhängigkeit von der Scherkraft ermittelt wird, bei dem die Haftreibung in die Gleitreibung übergangen ist. Dies kann beispielsweise anhand einer Tabelle erfolgen, bei dem für verschiedene Scherkräfte empirisch die jeweilige Klebrigkeit des Fasermaterials hinterlegt sind.

So ist es beispielsweise denkbar, dass die mechanische Messeinheit eine Auswerteeinheit hat, die in Abhängigkeit von der benötigten Scherkraft, bei der die Haftreibung in Gleitreibung übergeht, die Klebrigkeit des Fasermaterials ermittelt beziehungsweise berechnet.

Das Reibelement kann beispielsweise einen runden Querschnitt aufweisen und/oder rollen- beziehungsweise walzenförmig ausgebildet sein.

Das Reibelement der mechanischen Messeinheit kann darüber hinaus auch drehbar gelagert sein, um durch Erzeugen eines Drehmomentes im kontaktierten Zustand eine Scherkraft zwischen Fasermaterial und dem aufgedrückten Reibelement aufzubringen. Hierfür ist das Reibelement beispielsweise mit einem Motor verbunden, der gegebenenfalls über eine Getriebesteuerung zum Anlegen eines Drehmomentes an dem Reibelement ausgebildet ist. Über einen Drehsensor kann dann der Übergang von der Haftreibung zur Gleitreibung festgestellt werden, und zwar immer dann, wenn das Reibelement von einer nicht drehenden Bewegung in eine Drehbewegung übergeht. Die zu diesem Zeitpunkt aufgebrachte Scherkraft wird dann als Grundlage für die Ermittlung die Klebrigkeit verwendet. Der Drehsensor kann dabei auch zur Ermittlung des Drehmomentes ausgebildet sein, um so das aufgebrachte Drehmoment zum Zeitpunkt des Übergangs von der Haftreibung zur Gleitreibung feststellen zu können. Der Drehsensor kann somit auch ein Drehmomentsensor in einer Ausführungsform sein.

Denkbar ist allerdings auch, dass das Reibelement mit seiner gekrümmten Oberfläche feststehend und nicht drehbar ist und dass das kontaktierte Fasermaterial mit einer Krafteinheit so kommunizierend in Verbindung steht, dass über das Fasermaterial eine Scherkraft aufgebracht wird. Beim Übergang von der Haftreibung in die Gleitreibung würde sich dann das Fasermaterial gegenüber dem feststehenden Reibelement linear bewegen, so dass die zu diesem Zeitpunkt anliegende Scherkraft dann als Grundlage für die Ermittlung die Klebrigkeit verwendet wird. Auch hierbei kann mit Hilfe eines Momentsensors die aufgebrachte Scherkraft zum Zeitpunkt des Übergangs von der Haftreibung zur Gleitreibung festgestellt werden.

Erfindungsgemäß ist das Fasermaterial zwischen zwei voneinander beabstandet angeordneten Fixpunkte aufgespannt, wobei das Reibelement zwischen den beiden Fixpunkten auf das aufgespannte Fasermaterial mit der vorliegenden Kraft aufgedrückt wird. Vorteilhafterweise wird das Reibelement im Wesentlichen mittig zwischen den beiden Fixierpunkten auf das aufgespannte Fasermaterial gedrückt.

Darüber kann die Ermittlungseinrichtung zusätzlich eine optische Messeinheit aufweisen, die mindestens eine Kamera hat, die zum Aufnehmen von Bilddaten des Fasermaterials ausgebildet ist. Hierdurch können Bilddaten einer Fasermaterialoberfläche des Fasermaterials aufgenommen werden, wobei mithilfe einer Bildauswerteeinheit eine Oberflächeneigenschaft der Fasermaterialoberfläche aus den aufgenommenen Bilddaten ermittelt wird. Aus der ermittelten Oberflächeneigenschaft des Fasermaterials lässt sich dann mithilfe der optischen Messeinheit die Klebrigkeit des Fasermaterials ermitteln bzw. ableiten, so dass mit der optischen Messeinheit ein kontinuierliches und berührungsloses Erfassen der Klebrigkeit des Fasermaterials möglich wird.

Die Erfinder haben dabei erkannt, dass sich mit der Veränderung der Klebrigkeit auch eine oder mehrere Oberflächeneigenschaften der Fasermaterialoberfläche ändern, so dass durch die Ermittlung einer der Oberflächeneigenschaften Rückschlüsse auf die Klebrigkeit des Fasermaterials geführt werden können.

Dabei ist es denkbar, dass als Oberflächeneigenschaft die Reflexionseigenschaft der Fasermaterialoberfläche ermittelt wird. Die Reflexionseigenschaft im Sinne der vorliegenden Erfindung kann beispielsweise der Oberflächenglanz oder die Lichtstreuung sein. Es ist daher vorteilhafterweise so, dass die Bildauswerteeinheit der optischen Messeinheit eingerichtet ist, den Oberflächenglanz und/oder die Lichtstreuung aus den Bilddaten der aufgenommenen Fasermaterialoberfläche zu bestimmen und anhand des Oberflächenglanzes und/oder anhand der Lichtstreuung dann die Klebrigkeit des Fasermaterials ermittelt.

In einer weiteren Ausführungsform ist die Bildauswärteeinheit eingerichtet, die Farbe und/oder den Farbverlauf der Fasermaterialoberfläche aus den Bilddaten der aufgenommenen Fasermaterialoberfläche als Oberflächeneigenschaft zu bestimmen, um so anhand der Farbe und/oder des Farbverlaufes der Fasermaterialoberfläche die Klebrigkeit des Fasermaterials zu bestimmen. Denn die Erfinder haben hierbei erkannt, dass mit der Veränderung der Klebrigkeit des Fasermaterials und mit der Zunahme des Alters (Tacklife) letztendlich sich auch die Farbe bzw. der Farbverlauf auf der Fasermaterialoberfläche ändert. Diese kann je nach Fasermaterial und Hersteller empirisch für die verschiedenen Klebrigkeiten ermittelt und in einem Speicher hinterlegt sein.

In einer weiteren vorteilhaften Ausführungsform ist es auch denkbar, dass die optische Messeinheit eine Temperiereinrichtung hat, um die Fasermaterialoberfläche zu temperieren, und die Kamera eine Thermographiekamera ist, um so thermographische Bilddaten von der Fasermaterialoberfläche aufzunehmen. Die Bildauswärteeinheit ist nun so eingerichtet, dass aus den aufgenommenen thermographischen Bilddaten der Thermographiekamera die Oberflächentemperatur der Fasermaterialoberfläche als Oberflächeneigenschaft bestimmt wird, wobei die optische Messeinheit zum Ermitteln der Klebrigkeit des Fasermaterials in Abhängigkeit von der ermittelten Oberflächentemperatur und/oder eines von der ermittelten Oberflächentemperatur über die Zeit abgeleiteten Oberflächentemperaturverlaufs eingerichtet ist.

So ist es in dieser Ausführungsform beispielsweise denkbar, dass das Fasermaterial mit Hilfe der Temperiereinrichtung kurzzeitig erwärmt wird, wobei sowohl die Phase der Erwärmung als auch die anschließende Phase der Abkühlung des Fasermaterials mit Hilfe der Thermographiekamera aufgenommen wird und hieraus ein Oberflächentemperaturverlauf generiert wird. Aus diesem Oberflächentemperaturverlauf, der eine Aufheiz- und Abkühlkurve der Oberflächentemperatur abbildet, kann dann die Klebrigkeit des Fasermaterials bestimmt werden, da festgestellt wurde, dass mit zunehmender Tacklife sich ein derartiger Oberflächentemperaturverlauf verändert und die Aufheiz- und Abkühlkurve der Oberflächentemperatur somit für die Klebrigkeit des Materials und die Tacklife charakteristisch ist. Auch hier kann im Vorfeld für bestimmte Fasermaterialien und Fasermaterialhersteller entsprechend empirisch für verschiedene Klebrigkeiten bzw. Tacklife ein entsprechender Oberflächentemperaturverlauf erzeugt werden.

Es ist im Übrigen auch Aufgabe der vorliegenden Erfindung eine Faserlegevorrichtung anzugeben, mit der der Tack des Fasermaterials prozesssicher detektiert werden kann.

Gelöst wird die Aufgabe mit der Vorrichtung gemäß Anspruch 7 sowie dem Verfahren gemäß Anspruch 15.

Demnach ist eine Faserlegevorrichtung zum Legen von Fasermaterial auf einem Werkzeug zur Herstellung eines Faserverbundbauteils vorgesehen, wobei die Faserlegevorrichtung ein Fasermaterial zur Bereitstellung des Fasermaterials speichert und einen Bewegungsautomaten hat. Am Ende der kinematischen Kette des Bewegungsautomaten ist als Endeffektor ein Faserlegekopf vorgesehen, der das hinzugeführte Fasermaterial auf das Werkzeug legt. Hierfür ist der Bewegungsautomat so ausgebildet, dass er den Faserlegekopf gegenüber dem Werkzeug frei bewegen kann, sodass die Position sowie Anpresskraft mithilfe des Bewegungsautomaten vorgegeben werden kann.

Erfindungsgemäß ist nun vorgesehen, dass die Faserlegevorrichtung eine Ermittlungseinrichtung der vorstehend genannten Art zum Ermitteln die Klebrigkeit der abzulegenden Fasermaterialien aufweist.

So ist es beispielsweise denkbar, dass die mechanische Messeinheit so ausgebildet ist, dass sie im nicht ablegenden Zustand eine Messung durchführt, indem ein zwischen zwei Führungsrollen aufgespanntes Fasermaterial mit dem Reibelement bedruckt und anschließend die Messung durchgeführt wird.

Denkbar ist aber auch, dass die optische Messeinheit der Ermittlungseinrichtung beispielsweise im Faserkopf angeordnet ist und dort kontinuierlich die Klebrigkeit des Fasermaterials ermittelt. Denkbar ist natürlich auch, dass die optische Messeinheit an anderen Positionen der Faserführungskette angeordnet ist.

In einer vorteilhaften Ausführungsform ist es nunmehr möglich, dass die Steuereinheit der Faserlegevorrichtung zum Steuern des Ablegeprozesses so ausgebildet ist, dass der Ablegeprozess durch die Steuereinheit weiterhin auch in Abhängigkeit der ermittelten Klebrigkeit durchgeführt wird. So lassen sich beispielswiese aus der ermittelten Klebrigkeit die für den Ablegeprozess wichtige Prozessparameter wie beispielsweise Temperatur, Ablegegeschwindigkeit und/oder Andruckkraft anpassen, so dass das Fasermaterial hinsichtlich seiner aktuellen Klebrigkeit immer mit den optimalen Prozessparametern abgelegt wird. Die Erfindung wird anhand der beigefügten Figuren beispielhaft erläutert. Es zeigen:
- Fig. 1: schematische Darstellung einer Faserlegevorrichtung mit erfindungsgemäßer Ermittlungseinrichtung;
- Fig. 2: schematische Darstellung der mechanischen Messeinheit der erfindungsgemäßen Ermittlungseinrichtung;
- Fig. 3: schematische Darstellung der optischen Messeinheit der erfindungsgemäßen Ermittlungseinrichtung.

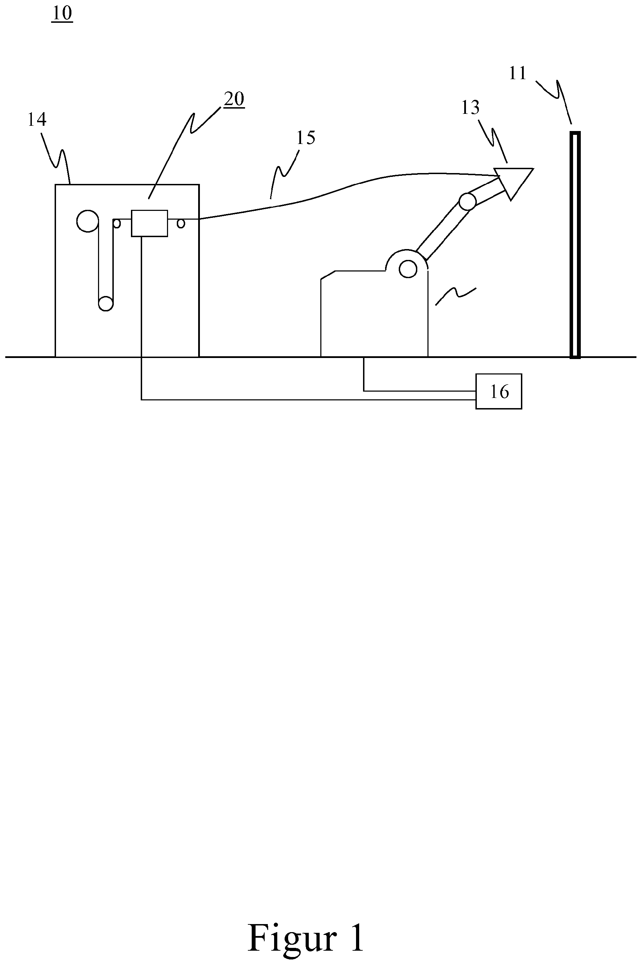

Figur 1 zeigt eine Faserlegevorrichtung 10 zum Legen von Fasermaterial auf einem Werkzeug 11. Bei der dargestellten Faserlegevorrichtung 10 in Figur 1 handelt es sich um eine automatisierte und robotergestützte Legevorrichtung. Die Faserlegevorrichtung 10 weist einen Bewegungsautomaten 12 auf, der in Form eines Knickarmroboters ausgebildet ist. An seinem einen Ende ist als Endeffektor ein Faserlegekopf 13 angeordnet, der mithilfe des Bewegungsautomaten 12 im Raum frei bewegbar und gegenüber dem feststehenden Werkzeug 11 verfahrbar ist.

Die Faserlegevorrichtung 10 weist des Weiteren einen feststehenden Fasermaterialspeicher 14 auf, dem Fasermaterial 15 bereitgestellt wird. Von dem Fasermaterialspeicher 14 wird dann das Fasermaterial 15 dem Faserlegekopf 13 zugeführt, damit es auf dem Werkzeug 11 entsprechend abgelegt werden kann, um so später ein Faserverbundbauteil herstellen zu können. Dabei wird das Fasermaterial 15 mithilfe einer nicht dargestellten Faserzuführvorrichtung von dem Fasermaterialspeicher 14 zu dem Faserlegekopf 13 kontinuierlich transportiert.

Der Bewegungsautomat beziehungsweise Roboter 12 ist des Weiteren mit einer Steuereinheit 16 verbunden, welche die Bewegung des Bewegungsautomaten 12 in Abhängigkeit der vorgegebenen Prozessparameter steuert. So kann insbesondere die Position sowie die Anpresskraft durch eine entsprechende Ansteuerung des Bewegungsautomaten 12 gesteuert werden, so dass das Fasermaterial an der vorgegebenen Position mit der vorgegebenen Anpresskraft auf dem Werkzeug 11 abgelegt wird. Durch eine entsprechende Ansteuerung lässt sich darüber hinaus auch die Ablegegeschwindigkeit des Fasermaterials 15 auf dem Werkzeug 11 steuern. Ist in dem Faserlegekopf 13 beispielsweise eine Heizeinrichtung vorgesehen, so lässt sich darüber hinaus auch die Temperatur des Fasermaterials 15 beim Ablegen auf das Werkzeug 11 mithilfe der Steuereinheit 16 steuern.

Erfindungsgemäß ist nun eine Ermittlungseinrichtung 20 vorgesehen, die zum Ermitteln der Klebrigkeit des vorimprägnierten Fasermaterials 15 ausgebildet ist. Im Ausführungsbeispiel der Figur 1 ist die Ermittlungseinrichtung 20 im Fasermaterialspeicher 14 vorgesehen. Denkbar ist selbstverständlich auch, dass die Ermittlungseinrichtung 20 in der Faserzuführung zwischen Fasermaterialspeicher 14 und Faserlegekopf 13 oder im Faserlegekopf 13 selber vorgesehen ist.

Die Ermittlungseinrichtung 20 der Faserlegefunktion 10 kann beispielsweise eine mechanische Messeinheit und/oder eine optische Messeinheit aufweisen um die Klebrigkeit des Fasermaterials zu ermitteln. Beide Messeinheiten sollen nun näher erläutert werden.

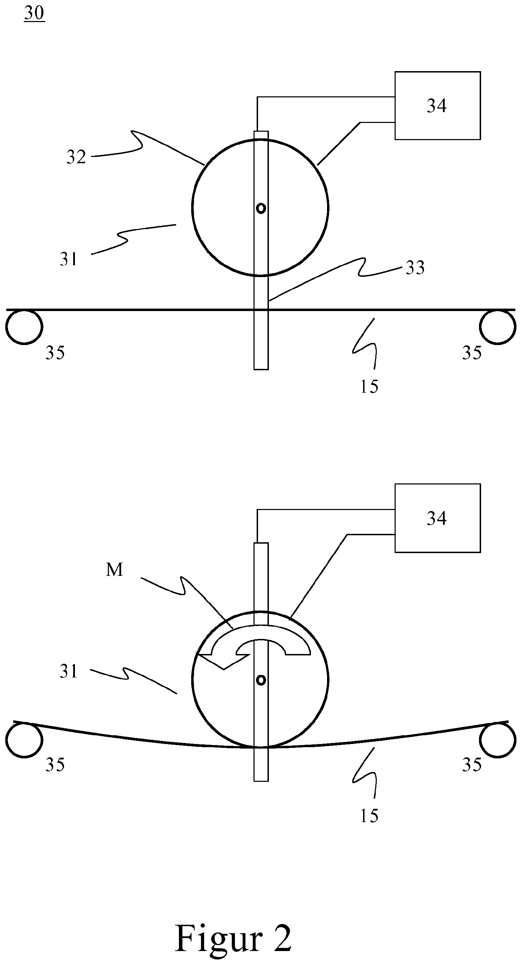

Figur 2 zeigt schematisch die optische Messeinheit 30 in einem nicht messenden Zustand (obere Abbildung) und in einem messenden Zustand (untere Abbildung). Die optische Messeinheit 30 weist ein Reibelement 31 auf, das eine gekrümmte Oberfläche 32 hat. Das Reibelement 31 ist im Ausführungsbeispiel der Figur 2 in Form einer Rolle oder Walze ausgebildet. Aufgehängt ist das Reibelement 31 an einer Linearverschiebung 33, mit der das Reibelement 31 in Bezug auf das Fasermaterial 15 bewegt werden kann. Darüber hinaus ist das Reibelement 31 drehbar vorgesehen, so dass auf das Reibelement 31 ein Drehmoment beaufschlagt werden kann. Sowohl die Linearverschiebung 33 als auch die drehbare Lagerung des Reibelement 31 sind mit einer Steuer- und Auswerteeinheit 34 verbunden, die zum Ansteuern der Linearverschiebung 33 zum Verschieben des Reibelement 31 sowie zum Anlegen eines Drehmomentes an das Reibelement 31 ausgebildet ist.

Das Fasermaterial, bei dem die Klebrigkeit gemessen werden soll, ist zwischen zwei Führungsrollen 35 aufgespannt, wobei zwischen den beiden Führungsrollen 35 das Reibelement linear verschiebbar angeordnet ist.

In der oberen Abbildung ist das Reibelement 31 in einer nicht messenden Position dargestellt, in der das Reibelement 31 keinen Kontakt zu dem Fasermaterial 15 hat. Das Fasermaterial 15 kann in diesem Zustand ungeändert über die Führungsrollen 35 transportiert und gefördert werden.

Soll nun eine Messung durchgeführt werden, so ist es vorteilhaft, wenn das Fasermaterial 15 nicht weiter gefördert wird und zwischen den beiden Führungsrollen 35 eingespannt ist. Mithilfe der Steuer- und Auswerteeinheit 34 wird nun das Reibelement in Richtung des Fasermaterials 15 gedrückt, so dass das Fasermaterial die gekrümmte Oberfläche 32 an einer vorgegebenen Länge der gekrümmten Oberfläche 32 kontaktiert, so wie in der Figur 2 (untere Abbildung) dargestellt wird. Anschließend wird an dem Reibelement 31 mithilfe der Steuer- und Auswerteeinheit 34 ein Drehmoment M angelegt, das kontinuierlich gesteigert wird. Mithilfe eines nicht dargestellten Drehsensors kann die Steuer- und Auswerteeinheit 34 feststellen, ob sich das Reibelement 31 dreht oder noch feststeht. In dem Augenblick, in dem das Drehmoment M eine Stärke erreicht hat, bei der die Haftreibung zwischen Reibelement 31 und dem Fasermaterial 15 in Gleitreibung übergeht, wird der Drehbeginn des Reibelementes 31 durch den Drehsensor detektiert und von der Steuer- und Auswerteeinheit 34 festgestellt, so dass das angelegte Drehmoment M, bei dem die Haftreibung in Gleitreibung übergegangen ist, als Basis für die Ermittlung des Tacks herangezogen wird. Denkbar ist auch, dass der Drehsensor auch ein Drehmomentsensor ist, der ein angelegtes Drehmoment ermittelt kann.

So kann beispielsweise aus einer empirisch ermittelten Tabelle ausgelesen werden, bei welchen maximalen Drehmomenten das Fasermaterial welchen Tack hat. So lassen sich aufgrund des ermittelten maximalen Drehmomentes, bei dem die Haftreibung noch besteht, der Tack des Fasermaterials ableiten.

Dabei wurde der Zusammenhang erkannt, dass bei einem niedrigeren Tack ein geringeres Drehmoment notwendig ist, um die Haftreibung aufzuheben und in die Gleitreibung überzugehen. Je älter beispielsweise das Fasermaterial ist, desto niedriger ist der Tack und desto geringer ist das notwendige Drehmoment, das benötigt wird, um die Haftreibung zu überwinden.

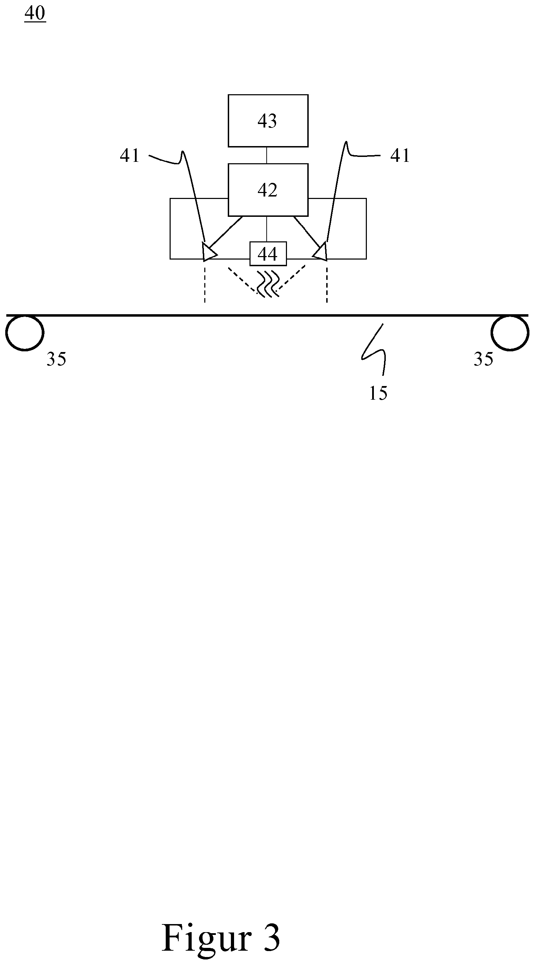

Figur 3 zeigt schematisch die optische Messeinheit 40, die zusätzlich Bestandteil der Ermittlungseinrichtung 20 sein kann. Die optische Messeinheit 40 im Ausführungsbeispiel der Figur 3 weist zwei Kameras 41 auf, die so ausgerichtet sind, dass sie das Fasermaterial 15 aus zwei verschiedenen Richtungen aufnehmen können. Die dabei aufgenommenen Bilddaten werden an eine Bildauswerteeinheit 42 übertragen, das beispielsweise ein Bildauswerteprogramm enthält und so die Reflexionseigenschaft oder die Farbe bzw. den Farbverlauf des Fasermaterials 15 aus den aufgenommenen Bilddaten ermitteln kann. Der Wert der Reflexionseigenschaft oder die Farbe bzw. der Farbverlaug, der von der Bildauswerteeinheit 42 aufgrund der aufgenommen Bilddaten ermittelt wird, wird dann an eine Auswerteeinheit 43 übertragen, die dann in Abhängigkeit der ermittelten Reflexionseigenschaft oder der Farbe bzw. des Farbverlaufes dann die Klebrigkeit des Fasermaterials 15 ermittelt.

So kann die Bildauswerteeinheit 42 beispielsweise den Oberflächenglanz und/oder die Lichtstreuung als Reflexionseigenschaft ermitteln, wobei dann die Auswerteeinheit entsprechend des Oberflächenglanzes und/oder der Lichtstreuung dahingehend ermittelt. Auch hier ist es denkbar, dass aus dem Parameter der Reflexionseigenschaft dann die Klebrigkeit aus einer Tabelle, die beispielsweise empirisch ermittelbar ist, ausgelesen wird.

Der Vorteil der optischen Messeinheit 40 liegt darin, dass das Fasermaterial 15 kontinuierlich weitergefördert werden kann, während der Tack des Fasermaterials ermittelt wird. Dadurch eignet sich die optische Messeinheit auch zur Anordnung im Faserlegekopf.

Denkbar ist auch, dass die beiden Kameras 41 Thermographiekameras sind, die thermographische Bilddaten der Faserhalbzeugoberfläche aufnehmen. Hierfür wird zunächst mit Hilfe einer Temperiereinrichtung 44 die Oberfläche temperiert und anschließend durch Ausschalten der Temperiereinrichtung 44 abgekühlt, so dass aus den aufgenommenen thermographischen Bilddaten dann ein Oberflächentemperaturverlauf der Oberfläche während der Temperierung und des Abkühlens ermittelt wird. Aus diesem Oberflächentemperaturverlauf lässt sich dann die Klebrigkeit des Fasermaterials ermitteln, da je nach Tacklife und somit Klebrigkeit des Fasermaterials eine charakteristische Aufheiz- und Abkühlkurve des Oberflächentemperaturverlaufes ergibt.

### Bezugszeichenliste

- 10: Faserlegevorrichtung
- 11: Werkzeug
- 12: Bewegungsautomat/Roboter
- 13: Faserlegekopf
- 14: Fasermaterialspeicher
- 15: Fasermaterial
- 16: Steuereinheit
- 20: Ermittlungseinrichtung
- 30: mechanische Messeinheit
- 31: Halbelement
- 32: gekrümmte Oberfläche
- 33: Linearverschiebung
- 34: Steuer- und Auswerteeinheit der mechanischen Messeinheit
- 35: Führungsrollen
- M: Drehmoment
- 40: optische Messeinheit
- 41: Kamera
- 42: Bildauswerteeinheit
- 43: Auswerteeinheit
- 44: Temperiereinrichtung

## Patentansprüche

1. Ermittlungseinrichtung (20) zum Ermitteln der Klebrigkeit von vorimprägnierten Fasermaterialen (15) für die Herstellung eines Faserverbundbauteils, **dadurch gekennzeichnet, dass** die Einrichtung (20) eine mechanische Messeinheit (30) aufweist, die mindestens ein eine gekrümmte Oberfläche (32) aufweisendes Reibelement (31) hat, das mit einer vorgegebenen Kraft mit der gekrümmten Oberfläche auf ein aufgespanntes Fasermaterial (15) aufdrückbar ist, sodass eine vorgegebene Länge der gekrümmten Oberfläche (32) mit dem Fasermaterial (15) kontaktiert, wobei die mechanische Messeinheit (30) zum Aufbringen einer Scherkraft (M) zwischen dem Fasermaterial (15) und dem aufgedrückten Reibelement (31) ausgebildet und zum Ermitteln der Klebrigkeit des kontaktierten Fasermaterials (15) in Abhängigkeit von der benötigten Scherkraft (M), bei dem die Haftreibung zwischen Fasermaterial (15) und gekrümmter Oberfläche (32) des Reibelements (31) in Gleitreibung übergeht, eingerichtet ist, wobei die mechanische Messeinheit (30) zwei voneinander beabstandete Fixierpunkte umfasst und dazu ausgebildet ist, das Fasermaterial (15) zwischen den Fixierpunkten (35) aufzuspannen und wobei das Reibelement (31) ausgebildet ist um zwischen den beiden Fixierpunkten auf das aufgespannte Fasermaterial mit der vorgegebenen Kraft aufgedrückt zu werden.

2. Ermittlungseinrichtung (20) nach Anspruch 1, **dadurch gekennzeichnet, dass** bei der mechanischen Messeinheit (30) das mindestens eine Reibelement (31) in Richtung des Fasermaterials (15) bewegbar gelagert ist, um das Reibelement (31) mit der gekrümmten Oberfläche (31) auf das aufgespannte Fasermaterial (15) zu drücken, und drehbar gelagert ist, um durch Erzeugen eines Drehmomentes (M) im kontaktierten Zustand eine Scherkraft zwischen dem Fasermaterial (15) und dem aufgedrückten Reibelement aufzubringen, wobei mittels eines Drehsensors der Übergang von der Haftreibung zur Gleitreibung feststellbar ist.

3. Ermittlungseinrichtung (20) nach Anspruch 1 oder 2, **dadurch gekennzeichnet, dass** bei der mechanischen Messeinheit (30) das mindestens eine Reibelement (31) einen runden Querschnitt aufweist und/oder das mindestens eine Reibelement rollen- oder walzenförmig ausgebildet ist.

4. Ermittlungseinrichtung (20) nach einem der vorhergehenden Ansprüche, **dadurch gekennzeichnet, dass** eine optische Messeinheit (40) vorgesehen ist, die mindestens eine Kamera (41) hat, die zum Aufnehmen von Bilddaten einer Fasermaterialoberfläche eines vorimprägnierten Fasermaterials (15) ausgebildet ist, und die eine Bildauswerteeinheit (42) hat, die zum Ermitteln einer Oberflächeneigenschaft der Fasermaterialoberfläche aus den aufgenommenen Bilddaten eingerichtet ist, wobei die optische Messeinheit (40) zum Ermitteln der Klebrigkeit des Fasermaterials (15) in Abhängigkeit von der ermittelten Oberflächeneigenschaft eingerichtet ist.

5. Ermittlungseinrichtung (20) nach Anspruch 4, **dadurch gekennzeichnet, dass** bei der optischen Messeinheit (40) die Bildauswerteeinheit (42) eingerichtet ist, die Reflexionseigenschaft, insbesondere den Oberflächenglanz und/oder die Lichtstreuung, aus den Bilddaten der aufgenommenen Fasermaterialoberfläche als Oberflächeneigenschaft zu bestimmen, und/oder die Farbe und/oder den Farbverlauf der Fasermaterialoberfläche aus den Bilddaten der aufgenommenen Fasermaterialoberfläche als Oberflächeneigenschaft zu bestimmen.

6. Ermittlungseinrichtung (20) nach Anspruch 4 oder 5, **dadurch gekennzeichnet, dass** die optische Messeinheit (40) eine Temperiereinrichtung hat, um die Fasermaterialoberfläche zu temperieren, und die Kamera (41) eine Thermografiekamera ist, wobei die Bildauswerteinheit (42) eingerichtet ist, aus den aufgenommenen Bilddaten der Thermografiekamera die Oberflächentemperatur der Fasermaterialoberfläche als Oberflächeneigenschaft zu bestimmen, wobei die optische Messeinheit zum Ermitteln der Klebrigkeit des Fasermaterials (15) in Abhängigkeit von der ermittelten Oberflächentemperatur und/oder eines von der ermittelten Oberflächentemperatur über die Zeit abgeleiteten Oberflächentemperaturverlaufes eingerichtet ist.

7. Faserlegevorrichtung (10) zum Legen von Fasermaterial (15) auf einem Werkzeug (11) zur Herstellung eines Faserverbundbauteils mit einem Fasermaterialspeicher (14) zur Bereitstellung des Fasermaterials (15), einem Bewegungsautomaten (12) und einem an dem Bewegungsautomaten (12) angeordneten Faserlegekopf (13), der zum Ablegen des aus dem Fasermaterialspeicher(14) zugeführten Fasermaterials (15) ausgebildet ist, **dadurch gekennzeichnet, dass** die Faserlegevorrichtung eine Ermittlungseinrichtung(20) zum Ermitteln der Klebrigkeit der abzulegenden Fasermaterialen nach einem der vorhergehenden Ansprüche aufweist.

8. Faserlegevorrichtung (10) nach Anspruch 7, **dadurch gekennzeichnet, dass** die Ermittlungseinrichtung (20) zum Ermitteln der Klebrigkeit in der Faserzuführung vor dem Faserlegekopf (13) oder in dem Faserlegekopf (13) angeordnet ist.

9. Faserlegevorrichtung (10) nach Anspruch 7 oder 8, **dadurch gekennzeichnet, dass** die Faserlegevorrichtung (10) eine Steuereinheit hat, die zum Steuern des Ablegeprozesses durch Ansteuerung des Bewegungsautomaten (12) in Abhängigkeit von der ermittelten Klebrigkeit des abzulegenden Fasermaterials (15) eingerichtet ist.

10. Verfahren zum Ermitteln der Klebrigkeit von vorimprägnierten Fasermaterialien für die Herstellung eines Faserverbundbauteils, **gekennzeichnet durch**
- Bereitstellen einer mechanischen Messeinheit (30), Aufdrücken eines eine gekrümmte Oberfläche aufweisendes Reibelementes mit einer vorgegebenen Kraft mit der gekrümmten Oberfläche (32) auf ein aufgespanntes Fasermaterial (15), sodass eine vorgegebene Länge der gekrümmten Oberfläche (12) mit dem Fasermaterial (15) kontaktiert, Aufbringen einer Scherkraft (M) zwischen dem Fasermaterial (15) und dem aufgedrückten Reibelement (31) und Ermitteln der Klebrigkeit des kontaktierten Fasermaterials (15) in Abhängigkeit von der benötigten Scherkraft (M), bei dem die Haftreibung zwischen Fasermaterial (15) und gekrümmter Oberfläche (32) des Reibelements (31) in Gleitreibung übergeht, mittels der mechanischen Messeinheit (30), wobei das Fasermaterial (15) zwischen zwei voneinander beabstandet angeordneten Fixpunkten aufgespannt wird und wobei das Reibelement (31) der mechanischen Messeinheit (30) zwischen den beiden Fixpunkten, insbesondere im Wesentlichen mittig, auf das aufgespannte Fasermaterial (15) mit der vorgegebenen Kraft aufgedrückt wird.

11. Verfahren nach Anspruch 10, **dadurch gekennzeichnet, dass** ein Drehmoment (M) am Reibelement (31) erzeugt wird, um eine Scherkraft (M) zwischen dem Fasermaterial (15) und dem aufgedrückten Reibelement (31) aufzubringen, und der Übergang von der Haftreibung zur Gleitreibung mittels eines Drehsensors festgestellt wird.

12. Verfahren nach einem der Ansprüche 10 bis 11, **gekennzeichnet durch** Bereitstellen einer optischen Messeinheit (40), Aufnehmen von Bilddaten einer Fasermaterialoberfläche eines vorimprägnierten Fasermaterials (15) mittels einer Kamera (41) der optischen Messeinheit (40), Ermitteln einer Oberflächeneigenschaft der Fasermaterialoberfläche aus den aufgenommenen Bilddaten mittels einer Bildauswerteeinheit (42) und Ermitteln der Klebrigkeit des Fasermaterials (15) in Abhängigkeit von der ermittelten Oberflächeneigenschaft mittels der optischen Messeinheit.

13. Verfahren nach Anspruch 12, **dadurch gekennzeichnet, dass** die Reflexionseigenschaft, insbesondere der Oberflächenglanz und/oder die Lichtstreuung, aus den Bilddaten der aufgenommenen Fasermaterialoberfläche als Oberflächeneigenschaft mittels der optischen Messeinheit (30) bestimmt wird und/oder die Farbe und/oder der Farbverlauf der Fasermaterialoberfläche aus den Bilddaten der aufgenommenen Fasermaterialoberfläche als Oberflächeneigenschaft mittels der optischen Messeinheit (30) bestimmt wird.

14. Verfahren nach Anspruch 12 oder 13, **dadurch gekennzeichnet, dass** mittels einer Temperiereinrichtung die Fasermaterialoberfläche temperiert wird und die Fasermaterialoberfläche mittels einer Thermografiekamera als Kamera aufgenommen wird, wobei aus den aufgenommenen Bilddaten der Thermografiekamera die Oberflächentemperatur der Fasermaterialoberfläche als Oberflächeneigenschaft mittels der Bildauswerteeinheit bestimmt und die Klebrigkeit des Fasermaterials (15) in Abhängigkeit von der ermittelten Oberflächentemperatur und/oder eines von der ermittelten Oberflächentemperatur über die Zeit abgeleiteten Oberflächentemperaturverlaufes mittels der optischen Messeinheit ermittelt wird.

15. Verfahren zum Legen von Fasermaterial (15) auf ein Werkzeug (11) zur Herstellung eines Faserverbundbauteils, bei dem ein in einem Fasermaterialspeicher (14) bereitgestelltes Fasermaterial (15) zu einem an einem Bewegungsautomaten (12) angeordneten Faserlegekopf (13) geführt wird, um das zugeführte Fasermaterial (15) durch den Faserlegekopf (13) auf das Werkzeug (12) zu legen, **dadurch gekennzeichnet, dass** die Klebrigkeit des abzulegenden Fasermaterials (15) mit dem Verfahren nach einem der Ansprüche 10 bis 14 ermittelt wird.

16. Verfahren nach Anspruch 15, **dadurch gekennzeichnet, dass** der Ablegeprozess durch Ansteuerung des Bewegungsautomaten (12) in Abhängigkeit von der ermittelten Klebrigkeit des abzulegenden Fasermaterials (15) mittels einer Steuereinheit gesteuert wird.

## Claims

1. Determining device (20) for determining the tackiness of pre-impregnated fibre materials (15) for the production of a fibre composite component, **characterized in that** the device (20) has a mechanical measuring unit (30), which has at least one friction element (31), which has a curved surface (32) and can be pressed by the curved surface onto a stretched fibre material (15) with a predetermined force, so that a predetermined length of the curved surface (32) comes into contact with the fibre material (15), wherein the mechanical measuring unit (30) is designed for applying a shearing force (M) between the fibre material (15) and the pressed-on friction element (31) and is set up for determining the tackiness of the contacted fibre material (15) in dependence on the required shearing force (M), in which the static friction between the fibre material (15) and the curved surface (32) of the friction element (31) is transformed into sliding friction, wherein the mechanical measuring unit (30) comprises two spaced-apart fixing points and is set up to stretch the fibre material (15) between the fixing points (35) and wherein the friction element (31) is designed to be pressed onto the stretched fibre material between the two fixing points with the predetermined force.

2. Determining device (20) according to Claim 1, **characterized in that** in the mechanical measuring unit (30) the at least one friction element (31) is mounted movably in the direction of the fibre material (15), in order to press the friction element (31) by the curved surface (31) onto the stretched fibre material (15), and is mounted rotatably in order to apply a shearing force between the fibre material (15) and the pressed-on friction element by producing a torque (M) in the contacted state, wherein the transformation from the static friction to the sliding friction can be detected by means of a rotary sensor.

3. Determining device (20) according to Claim 1 or 2, **characterized in that** in the mechanical measuring unit (30) the at least one friction element (31) has a round cross section and/or the at least one friction element is designed in the form of a roller or a roll.

4. Determining device (20) according to one of the preceding claims, **characterized in that** an optical measuring unit (40) is provided, which has at least one camera (41) which is designed for recording image data of a fibre-material surface of a pre-impregnated fibre material (15), and which has an image evaluation unit (42), which is set up for determining a surface property of the fibre-material surface from the recorded image data, wherein the optical measuring unit (40) is designed for determining the tackiness of the fibre material (15) in dependence on the determined surface property.

5. Determining device (20) according to Claim 4, **characterized in that** in the optical measuring unit (40) the image evaluation unit (42) is set up to determine the reflection property, in particular the surface lustre and/or the light diffusion, from the image data of the recorded fibre-material surface as a surface property, and/or the colour and/or the variation in colour of the fibre-material surface from the image data of the recorded fibre-material surface as a surface property.

6. Determining device (20) according to Claim 4 or 5, **characterized in that** the optical measuring unit (40) has a temperature-controlling device, in order to control the temperature of the fibre-material surface, and the camera (41) is a thermal camera, wherein the image evaluation unit (42) is set up to determine the surface temperature of the fibre-material surface from the recorded image data of the thermal camera as a surface property, wherein the optical measuring unit is set up for determining the tackiness of the fibre material (15) in dependence on the determined surface temperature and/or a variation in surface temperature derived from the determined surface temperature over time.

7. Fibre laying apparatus (10) for laying fibre material (15) on a tool (11) for producing a fibre composite component, with a fibre material store (14) for providing the fibre material (15), a movement machine (12) and a fibre laying head (13), which is arranged on the movement machine (12) and is designed for laying down the fibre material (15) fed from the fibre material store (14), **characterized in that** the fibre laying apparatus has a determining device (20) for determining the tackiness of the fibre materials to be laid down according to one of the preceding claims.

8. Fibre laying apparatus (10) according to Claim 7, **characterized in that** the determining device (20) for determining the tackiness is arranged in the fibre feed upstream of the fibre laying head (13) or in the fibre laying head (13).

9. Fibre laying apparatus (10) according to Claim 7 or 8, **characterized in that** the fibre laying apparatus (10) has a control unit, which is set up for controlling the laying-down process by activating the movement machine (12) in dependence on the determined tackiness of the fibre material (15) to be laid down.

10. Method for determining the tackiness of pre-impregnated fibre materials for the production of a fibre composite component, **characterized by**
- providing a mechanical measuring unit (30), pressing a friction element having a curved surface by the curved surface (32) onto a stretched fibre material (15) with a predetermined force, so that a predetermined length of the curved surface (12) comes into contact with the fibre material (15), applying a shearing force (M) between the fibre material (15) and the pressed-on friction element (31) and determining the tackiness of the contacted fibre material (15) in dependence on the required shearing force (M) , in which the static friction between the fibre material (15) and the curved surface (32) of the friction element (31) is transformed into sliding friction, by means of the mechanical measuring unit (30), wherein the fibre material (15) is stretched between two fixed points arranged spaced apart from one another, and wherein the friction element (31) of the mechanical measuring unit (30) is pressed onto the stretched fibre material (15) between the two fixed points, in particular substantially centrally, with the predetermined force.

11. Method according to Claim 10, **characterized in that** a torque (M) is produced on the friction element (31) in order to apply a shearing force (M) between the fibre material (15) and the pressed-on friction element (31), and the transformation from the static friction to the sliding friction is detected by means of a rotary sensor.

12. Method according to either of Claims 10 and 11, **characterized by** providing an optical measuring unit (40), recording image data of a fibre-material surface of a pre-impregnated fibre material (15) by means of a camera (41) of the optical measuring unit (40), determining a surface property of the fibre-material surface from the recorded image data by means of an image evaluation unit (42) and determining the tackiness of the fibre material (15) in dependence on the determined surface property by means of the optical measuring unit.

13. Method according to Claim 12, **characterized in that** the reflection property, in particular the surface lustre and/or the light diffusion, is determined from the image data of the recorded fibre-material surface as a surface property by means of the optical measuring unit (30) and/or the colour and/or the variation in colour of the fibre-material surface is determined from the image data of the recorded fibre-material surface as a surface property by means of the optical measuring unit (30).

14. Method according to Claim 12 or 13, **characterized in that** the temperature of the fibre-material surface is controlled by means of a temperature-controlling device and the fibre-material surface is recorded by means of a thermal camera as a camera, wherein the surface temperature of the fibre-material surface is determined from the recorded image data of the thermal camera as a surface property by means of the image evaluation unit, and the tackiness of the fibre material (15) is determined in dependence on the determined surface temperature and/or a variation in surface temperature derived from the determined surface temperature over time by means of the optical measuring unit.

15. Method for laying fibre material (15) onto a tool (11) for producing a fibre composite component, in which a fibre material (15) provided in a fibre material store (14) is guided to a fibre laying head (13), arranged on a movement machine, in order to lay the fed fibre material (15) onto the tool (12) by the fibre laying head (13), **characterized in that** the tackiness of the fibre material (15) to be laid down is determined by the method according to one of Claims 10 to 14.

16. Method according to Claim 15, **characterized in that** the laying-down process is controlled by activating the movement machine (12) in dependence on the determined tackiness of the fibre material (15) to be laid down by means of a control unit.

## Revendications

1. Dispositif de détermination (20) pour déterminer la pégosité de matériaux fibreux pré-imprégnés (15) pour la fabrication d'un composant composite fibreux,
**caractérisé en ce que** le dispositif (20) comprend une unité de mesure mécanique (30) ayant au moins un élément de friction (31) qui présente une surface incurvée (32) et peut être pressé, avec une force prédéfinie, avec la surface incurvée sur un matériau fibreux tendu (15), de sorte qu'une longueur prédéfinie de la surface incurvée (32) entre en contact avec le matériau fibreux (15), l'unité de mesure mécanique (30) étant réalisée pour appliquer une force de cisaillement (M) entre le matériau fibreux (15) et l'élément de friction pressé (31) et étant conçue pour déterminer la pégosité du matériau fibreux (15) mis en contact, en fonction de la force de cisaillement requise (M) à laquelle le frottement statique entre le matériau fibreux (15) et la surface incurvée (32) de l'élément de friction (31) se transforme en frottement dynamique, l'unité de mesure mécanique (30) comprenant deux points de fixation espacés l'un de l'autre et étant réalisée pour tendre le matériau fibreux (15) entre les points de fixation (35), et l'élément de friction (31) étant réalisé pour être pressé, avec la force prédéfinie, sur le matériau fibreux tendu entre les deux points de fixation.

2. Dispositif de détermination (20) selon la revendication 1,
**caractérisé en ce que**, dans l'unité de mesure mécanique (30), ledit au moins un élément de friction (31) est monté de manière à pouvoir être déplacé en direction du matériau fibreux (15), afin de presser l'élément de friction (31) avec la surface incurvée (31) sur le matériau fibreux tendu (15), et est monté de manière à pouvoir tourner, afin d'appliquer une force de cisaillement entre le matériau fibreux (15) et l'élément de friction pressé en générant un couple (M) à l'état de contact, la transition du frottement statique au frottement dynamique pouvant être détectée au moyen d'un capteur de rotation.

3. Dispositif de détermination (20) selon la revendication 1 ou 2,
**caractérisé en ce que**, dans l'unité de mesure mécanique (30), ledit au moins un élément de friction (31) présente une section transversale ronde et/ou ledit au moins un élément de friction est réalisé en forme de rouleau ou de cylindre.

4. Dispositif de détermination (20) selon l'une des revendications précédentes,
**caractérisé en ce qu'**il est prévu une unité de mesure optique (40) ayant au moins une caméra (41) qui est conçue pour enregistrer des données d'image d'une surface d'un matériau fibreux pré-imprégné (15), et ayant une unité d'évaluation d'image (42) qui est conçue pour déterminer une propriété de surface de la surface de matériau fibreux à partir des données d'image enregistrées, l'unité de mesure optique (40) étant conçue pour déterminer la pégosité du matériau fibreux (15) en fonction de la propriété de surface déterminée.

5. Dispositif de détermination (20) selon la revendication 4,
**caractérisé en ce que**, dans l'unité de mesure optique (40), l'unité d'évaluation d'image (42) est conçue pour définir, en tant que propriété de surface, la propriété de réflexion, en particulier la brillance de la surface et/ou la diffusion de la lumière, à partir des données d'image de la surface enregistrée du matériau fibreux, et/ou pour définir, en tant que propriété de surface, la couleur et/ou le dégradé de couleur de la surface du matériau fibreux à partir des données d'image de la surface enregistrée du matériau fibreux.

6. Dispositif de détermination (20) selon la revendication 4 ou 5,
**caractérisé en ce que** l'unité de mesure optique (40) comprend un dispositif de régulation de la température pour réguler la température de la surface du matériau fibreux, et la caméra (41) est une caméra thermographique, l'unité d'évaluation d'image (42) étant conçue pour définir, en tant que propriété de surface, la température de surface de la surface du matériau fibreux à partir des données d'image enregistrées de la caméra thermographique, l'unité de mesure optique étant conçue pour déterminer la pégosité du matériau fibreux (15) en fonction de la température de surface déterminée et/ou d'une courbe de température de surface dérivée de la température de surface déterminée sur le temps.

7. Dispositif de pose de fibres (10) pour la pose de matériau fibreux (15) sur un outil (11) pour la fabrication d'un composant composite fibreux, comportant un réservoir de matériau fibreux (14) pour la mise à disposition du matériau fibreux (15), un automate de déplacement (12) et une tête de pose de fibres (13) disposée sur l'automate de déplacement (12), qui est conçue pour déposer le matériau fibreux (15) amené depuis le réservoir de matériau fibreux (14),
**caractérisé en ce que** le dispositif de pose de fibres comprend un dispositif de détermination (20) pour déterminer la pégosité des matériaux fibreux à déposer, selon l'une des revendications précédentes.

8. Dispositif de pose de fibres (10) selon la revendication 7,
**caractérisé en ce que** le dispositif de détermination (20) pour déterminer la pégosité est disposé dans l'alimentation en fibres avant la tête de pose de fibres (13) ou dans la tête de pose de fibres (13).

9. Dispositif de pose de fibres (10) selon la revendication 7 ou 8,
**caractérisé en ce que** le dispositif de pose de fibres (10) comprend une unité de commande qui est conçue pour commander le processus de pose en pilotant l'automate de déplacement (12) en fonction de la pégosité déterminée du matériau fibreux (15) à déposer.

10. Procédé pour déterminer la pégosité de matériaux fibreux pré-imprégnés pour la fabrication d'un composant composite fibreux, **caractérisé par** les étapes consistant à
mettre à disposition une unité de mesure mécanique (30), presser, avec une force prédéfinie, un élément de friction présentant une surface incurvée avec la surface incurvée (32) sur un matériau fibreux tendu (15), de sorte qu'une longueur prédéfinie de la surface incurvée (12) entre en contact avec le matériau fibreux (15),
appliquer une force de cisaillement (M) entre le matériau fibreux (15) et l'élément de friction (31) pressé, et déterminer la pégosité du matériau fibreux (15) mis en contact, en fonction de la force de cisaillement requise (M) à laquelle le frottement statique entre le matériau fibreux (15) et la surface incurvée (32) de l'élément de friction (31) se transforme en frottement dynamique, au moyen de l'unité de mesure mécanique (30),
le matériau fibreux (15) étant tendu entre deux points fixes espacés l'un de l'autre, et l'élément de friction (31) de l'unité de mesure mécanique (30) étant pressé, avec la force prédéfinie, sur le matériau fibreux tendu (15) entre les deux points fixes, en particulier sensiblement au centre.

11. Procédé selon la revendication 10,
**caractérisé en ce qu'**un couple (M) est généré sur l'élément de friction (31) afin d'appliquer une force de cisaillement (M) entre le matériau fibreux (15) et l'élément de friction (31) pressé, et la transition du frottement statique au frottement dynamique est détectée au moyen d'un capteur de rotation.

12. Procédé selon l'une des revendications 10 à 11,
**caractérisé par** les étapes consistant à mettre à disposition une unité de mesure optique (40), enregistrer des données d'image d'une surface d'un matériau fibreux pré-imprégné (15) au moyen d'une caméra (41) de l'unité de mesure optique (40), déterminer une propriété de surface de la surface du matériau fibreux au moyen d'une unité d'évaluation d'image (42) à partir des données d'image enregistrées, et déterminer la pégosité du matériau fibreux (15) en fonction de la propriété de surface déterminée, au moyen de l'unité de mesure optique.

13. Procédé selon la revendication 12,
**caractérisé en ce que** la propriété de réflexion, en particulier la brillance de la surface et/ou la diffusion de la lumière, est définie en tant que propriété de surface à partir des données d'image de la surface enregistrée du matériau fibreux, au moyen de l'unité de mesure optique (30), et/ou
la couleur et/ou le dégradé de couleur de la surface du matériau fibreux est défini(e) en tant que propriété de surface à partir des données d'image de la surface enregistrée du matériau fibreux, au moyen de l'unité de mesure optique (30).

14. Procédé selon la revendication 12 ou 13,
**caractérisé en ce que** la surface du matériau fibreux est régulée en température au moyen d'un dispositif de régulation de température, et la surface du matériau fibreux est enregistrée au moyen d'une caméra thermographique servant de caméra, la température de surface de la surface du matériau fibreux étant définie en tant que propriété de surface au moyen de l'unité d'évaluation d'image à partir des données d'image enregistrées de la caméra thermographique, et la pégosité du matériau fibreux (15) étant déterminée au moyen de l'unité de mesure optique en fonction de la température de surface déterminée et/ou d'une courbe de température de surface dérivée de la température de surface déterminée sur le temps.

15. Procédé de pose d'un matériau fibreux (15) sur un outil (11) pour la fabrication d'un composant composite fibreux, dans lequel un matériau fibreux (15) mis à disposition dans un réservoir de matériau fibreux (14) est amené à une tête de pose de fibres (13) disposée sur un automate de déplacement (12), afin de déposer le matériau fibreux amené (15) sur l'outil (12) par la tête de pose de fibres (13),
**caractérisé en ce que** la pégosité du matériau fibreux (15) à déposer est déterminée par le procédé selon l'une des revendications 10 à 14.

16. Procédé selon la revendication 15,
**caractérisé en ce que** le processus de pose est commandé au moyen d'une unité de commande en pilotant l'automate de déplacement (12) en fonction de la pégosité déterminée du matériau fibreux (15) à déposer.
